# EUROPEAN PATENT SPECIFICATION

(11) **EP 1 967 170 B1**
(45) Date of publication and mention of the grant of the patent: **06.11.2013**
(21) Application number: 06745657.4
(22) Date of filing: 25.04.2006
(51) Int. Cl.: A61F 13/00, A61F 13/02, A61K 9/70

(54) **ADHESIVE PATCH**
KLEBEPFLASTER
TIMBRE ADHESIF

(30) Priority: 28.12.2005 JP 2005377553
(43) Date of publication of application: 10.09.2008
(73) Proprietor: TEIKOKU SEIYAKU CO., LTD., Higashikagawa-shi, Kagawa 769-2695 (JP)
(72) Inventor: UEMATSU, Masanori, Kagawa 7612300 (JP)
(74) Representative: Albrecht, Thomas
(86) International application number: PCT/JP2006/308629
(87) International publication number: WO 2007/077639

(56) References cited:
- EP-A1- 0 360 458
- EP-A1- 0 630 629
- EP-A1- 1 170 006
- EP-A2- 0 434 258
- WO-A1-02/072081
- FR-A1- 2 794 969
- JP-A- 4 193 826
- JP-A- 07 500 751
- JP-A- 09 154 872
- JP-A- 11 501 224
- JP-A- 2000 219 622
- JP-B2- 3 192 333
- US-A1- 2002 086 043
- US-A1- 2005 232 982

## Description

### TECHNICAL FIELD

The present invention relates to adhesive patches, such as poultices, for the treatment of inflammation, pain, itch and other symptoms. More particularly, the present invention relates to an efficient medicated patch that can be easily applied not only by non-disabled people, but also by aged people and patients with decreased grip strength, by facilitating the sequence of actions required to apply the patch, from the removal of the peelable film (liner) to the application of the patch. The adhesive patch of the present invention can be applied in a safe and hygienic manner since the drug-containing matrix does not come into contact with hands during the application.

### BACKGROUND ART

Anti-inflammatory and analgesic poultices are now widely used in the conservative treatment of lumbago, joint pain, shoulder stiffness and other symptoms caused by aging or hard working. Adhesive patches are also widely used in the treatment of herpes zoster in recent years.

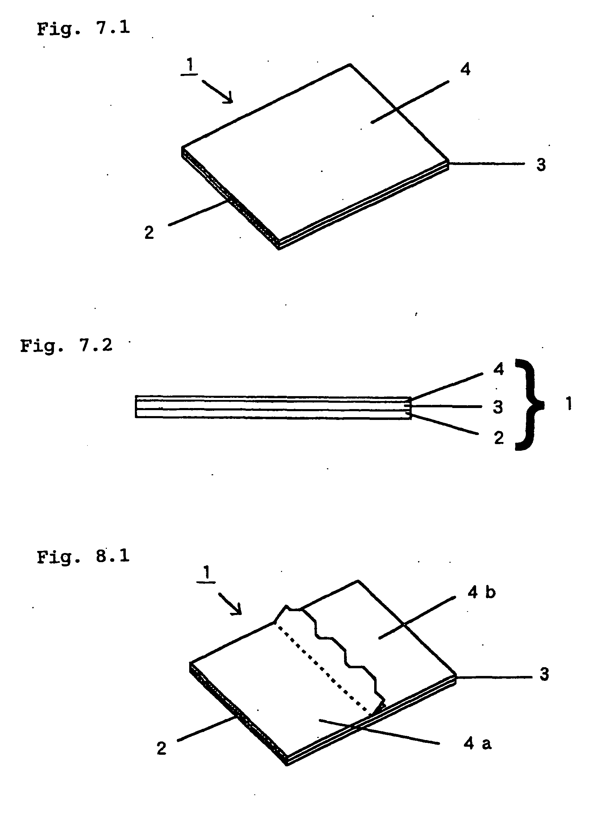

Conventional adhesive patches, such as poultice-type patches, typically have a multilayer structure. As an example, a conventional adhesive patch 1 is shown in Fig. 7. The adhesive patch 1 has a multilayer structure including a white or skin-colored backing 2, an adhesive drug-containing matrix 3 that is spread substantially entirely over one surface of the backing, and a sheet of liner 4 that entirely covers the drug-containing matrix.

Typically, such a adhesive patch (such as a poultice-type patch) is applied in the following manner: First, the user rubs the edge of the poultice-type patch with a substantial force to cause the liner 4 to slide relative to the surface of the drug-containing matrix 3. This causes the film to partially come off the surface of the matrix. The user quickly holds the drug-containing matrix with one finger before the film sticks again to the matrix and, using another finger, carefully removes the liner 4 so that the adhesive matrix will not stick to itself. Once the liner 4 is completely peeled, the user, holding the drug-containing matrix and the backing, applies to the application site.

A problem with these adhesive patches in which a single sheet of liner is used to cover the entire surface of the drug-containing matrix is that users often have difficulty applying these patches to the application site since the liner of these patches is difficult to remove from the surface of the matrix, often resulting in the matrix sticking to itself.

One proposed solution to this problem is an adhesive patch shown in Fig. 8 (Patent Document 1). The adhesive patch 1 includes a white or skin-colored backing 2, an adhesive drug-containing matrix 3 that is spread substantially entirely over one surface of the backing, and a pair of liners, an upper liner 4a and a lower liner 4b, that adheres to the drug-containing matrix.

To apply this adhesive patch, the user first pinches the tab of the upper liner 4a with his fingers and carefully removes the film 4a. The user then pinches the tab of the lower liner 4b with his fingers and carefully removes the film 4b so that the adhesive drug-containing matrix will not stick to itself. Once the film is completely peeled, the user, holding the drug-containing matrix and the backing, applies the patch to the application site.

Another type of adhesive patch, such as one shown in Fig. 9, is also proposed (See, for example, Patent Document 2). This adhesive patch 1 includes a white or skin-colored backing 2, an adhesive drug-containing matrix 3 that is spread substantially entirely over one surface of the backing, and a single liner 4 that has a perforation 5 substantially at the middle thereof and covers the entire surface of the drug-containing matrix.
To apply this adhesive patch, the user may pull apart the ends of the poultice-type patch to tear the single liner 4 along the perforation 5. The portion of the drug-containing matrix that is extended and exposed between the separated halves of the film is then attached to the application site and the separated halves of the film are peeled while the patch is pressed from above the backing.

Patent Document 1 Japanese Laid-Open Patent Publication No. 2001-219622
Patent Document 2 Japanese Patent No. 3192333

The above-described conventional adhesive patches, such as poultice-type patches, have the following problems that have yet to be addressed:
(1) The adhesive patches, such as poultice-type patches, are fabricated by spreading a drug-containing matrix substantially entirely over one surface of a backing, and placing a liner over the drug-containing matrix to form a laminate, or an original sheet, which is then cut into a desired shape, such as rectangular. In these patches, the backing, the drug-containing matrix and the liner are laminated together with their outer edges lying along the same plane. The patches therefore have no part along the outer edge that the user can hold to start peeling the liner. Thus, in order to peel the liner, the user must rub the patch with his fingers along its outer edge. This requires a certain type of dexterity and cannot readily be accomplished by aged people or other people with decreased dexterity.
(2) The drug-containing matrix is made to be highly sticky so that it can firmly stick to the application site. Thus, peeling the liner from the patch requires a substantial force. The adhesive patch, such as poultice-type patch, designed to be pulled by the ends to break and remove the liner requires an even greater force, making the patch unsuitable for use by aged people.
(3) All of these adhesive patches, such as poultice-type patches, are designed to be applied to the application site either after the liner has been entirely removed or while the film is being removed. Thus, the drug-containing matrix may come into direct contact with the fingers, which often results in the drug-containing matrix sticking to itself or wrinkled. To avoid this and to ensure proper application of the patch, the user must carefully and slowly apply the patch to the application site.
(4) The drug-containing matrix that comes into direct contact with the fingers during the peeling of the liner loses the initial stickiness and, as a result, the once-applied adhesive patch such as poultice-type patch may partially come off the application site.
(5) The drug-containing matrix that comes into direct contact with the fingers is unfavorable from the hygienic point of view and requires much attention when applied to the damaged skin.
(6) The active ingredient and other components present in the drug-containing matrix may be transferred to the finger tips through the direct contact with the drug-containing matrix. The transferred drug-containing matrix is sticky and causes discomfort and must therefore be removed by rinsing.
EP 0360 458 discloses an adhesive dressing having two liners, one being folded.

### DISCLOSURE OF THE INVENTION

### PROBLEMS TO BE SOLVED BY THE INVENTION

The present invention addresses the above-described problems of the conventional adhesive patches. To that end, it is an object of the present invention to provide a adhesive patch that can be easily applied not only by non-disabled people, but also by aged people and patients with decreased grip strength, by facilitating the sequence of actions required to apply the patch, from the removal of the liner to the application of the patch. The adhesive patch of the present invention can be applied in a safe and hygienic manner since the drug-containing matrix does not come into contact with hands during the application.

### MEANS FOR SOLVING THE PROBLEMS

One essential aspect of the present invention to solve the aforementioned problems comprises the following:
(1) an adhesive patch having a multilayer structure comprising a backing, an adhesive drug-containing matrix that is spread substantially entirely over one surface of the backing, and a liner that adheres to the drug-containing matrix surface, wherein the adhesive drug-containing matrix comprises sodium polyacrylate as thickener wherein
   (a) the liner adhering to the drug-containing matrix surface comprises first and second liners;
   (b) the first liner is folded at the middle thereof so that it is divided by the fold into first and second sections that together form a V-shaped liner, the first section adhering to the drug-containing matrix surface from one end of the matrix surface with the fold arranged closer to the middle of the drug-containing matrix, the second section serving as a tab of the V-shaped liner; wherein the tab of the first liner and/or second liner has an end shaped as a curved line, such as a wave shape or a mountain shape, or a combination thereof and
   (c) the second liner adheres to the remaining part of the drug-containing matrix surface with one end of the second liner covering the fold of the V-shaped first liner to form a laminated part that serves as a tab.

More specifically, the present invention comprises the following:
(2) the adhesive patch according to (1) above, wherein a ratio of an area of the drug-containing matrix surface to which the first liner adheres, to an area to which the second liner adheres is in a range of 3:1 to 1:3;
(3) the adhesive patch according to (1) above, wherein a ratio of an area of the drug-containing matrix surface to which the first liner adheres, to an area to which the second liner adheres is in a range of 1:1 to 2:1;
(4) the adhesive patch according to any of (1) to (3) above, wherein the tab of the second liner is 10 mm to 30 mm;
(5) the adhesive patch according to any of (1) to (4) above, wherein the tab of the first liner and/or the second liner has an end shaped as a straight line, a curved line, such as a wave shape or a mountain shape, or a combination thereof;
(6) the adhesive patch according to any of (1) to (5) above, wherein the first liner and/or the second liner comprises a plastic film formed of cast polypropylene, oriented polypropylene, polyethylene terephthalate, polybutylene terephthalate, polyethylene, polyester, polyurethane, polyvinyl chloride or polystyrene; paper, synthetic paper, synthetic resin or a composite film formed of a laminate thereof; or a composite film formed of a laminate of aluminum foil or aluminum-deposited film;
(7) the adhesive patch according to any of (1) through (6) above, wherein the first liner and/or the second liner is treated with silicone;
(8) the adhesive patch according to any of (1) through (7) above, wherein the first liner and/or the second liner is embossed;
(9) the adhesive patch according to any of (1) through (8) above, wherein the tab of the first liner and/or the second liner has a decorative pattern or is colored; and
(10) the adhesive patch according to any of (1) through (8) above, wherein the tab of the first liner and/or the second liner is embossed differently from the remaining part of the film.

### EFFECT OF THE INVENTION

As described above, one feature of the adhesive patch provided in accordance with the present invention is that two liners, first and second liners, adhere to the surface of the drug-containing matrix and each include a tab.
The user can easily pinch the tab of the upper second liner with his fingers and peel the film. This can be done in a safe and simple manner and requires only a weak force.
The user then pinches with his fingers the tab of the V-shaped lower first liner laminated to the backing and pulls the tab so that it slides outward. This causes the exposed drug-containing matrix to spontaneously attach to the application site.

Thus, by using the adhesive patch of the present invention, the user does not need to exert a substantial force to form a part from which to start peeling the liner upon removal of the film. The adhesive patch of the present invention therefore offers an advantage in that it enables people who cannot exert much physical power, such as aged people, to peel the liner. The adhesive patch thus facilitates the sequence of actions required to apply the patch, from the removal of the liner to the application of the patch with one hand.

The adhesive patch offers another advantage in that the patch can be applied without being deformed and the incidence of failed application is significantly reduced since the removal of the liner does not require forcibly pulling apart the ends of the adhesive patch.

Still another advantage of the adhesiva patch that uses a liner that can be removed without forcibly pulling apart the ends of the patch is that readily removable and applicable patches can be produced using non-stretching backings.

Still another advantage of the adhesive patch is that the stickiness and the skin followability of the drug-containing matrix are not affected since the drug-containing matrix does not come into contact with the hands or fingers during the sequence of actions from the removal of the liner to the application of the patch to the application site. Since the drug-containing matrix is free of contamination and maintains good hygiene, the adhesive patch is safe for application to the damaged skin.
Yet another advantage of the adhesive patch is that the patch does not cause sticky fingers or hands, so that good hygiene can be maintained during the application of the patch and the user does not need to wash hands after application of the patch.

### BEST MODE FOR CARRYING OUT THE INVENTION

The liner for use in the adhesive patch of the present invention may be a resin film formed of such as cast polypropylene (CPP), oriented polypropylene (OPP), polyethylene terephthalate (PET), polybutylene terephthalate (PBT), polyethylene, polyester, polyurethane, polyvinyl chloride and polystyrene, paper, synthetic paper, synthetic resin or a composite film formed of a laminate thereof, an aluminum foil or aluminum-deposited film laminated with any of the above-described materials, or each or a composite of the above-described materials that is treated with silicone, is embossed, has a printed pattern or is colored.

The liner has a thickness in the range of 10 to 200 µm, and preferably in the range of 25 to 75 pm. The liner with a thickness of less than 10 pm is too thin to be properly held by the fingers and tends to wrinkle during the production of the adhesive patches. The liner with a thickness of more than 200 µm is difficult to cut during the production of the adhesive patches, which adds to the cost of the adhesive patches. Such liner is also expensive and is therefore unfavorable in terms of cost.

According to the present invention, the liner is preferably embossed to prevent slipping of the fingers upon removal of the film. The liner may be embossed either entirely or partially (for example, in the tab).

The liner may be embossed with any pattern that provides a good grip for the fingers, such as diamond pattern, lattice pattern, hexagonal pattern, wave pattern and various other patterns.

To clearly indicate to the user the manner of peeling the liner, characters, arrows, symbols, illustrations and other indicative marks may be provided on either or both of the first and second liners. Either or both of the first and second liners may also be colored.

The part that serves as the tab of the second liner, that is, the overlap (laminated part) of the upper liner and the lower V-shaped first liner, is preferably from about 10 to 30mm wide, and more preferably from 15 to 25mm wide. The tab that is less than 10mm wide is not desirable since it can hardly be held by the fingers and cause meandering during production, thus leading to a decreased workability. It also leads to a decreased yield of the adhesive patches. The tab that is more than 30mm wide is not desirable, either, since the laminated part not only adds to the cost, but also tends to roll up upon packaging, though the wider tab is easier to hold with the fingers.

Preferably, the laminated part of the upper second liner and the lower V-shaped first liner may contain characters, arrows, symbols, illustrations and other indicative marks that indicate the end to the user. The laminated part may also be colored for the same purpose. To further distinguish the tab, the tab may be embossed differently from the remaining part of the film.

The upper second liner and the lower V-shaped first liner may adhere to the drug-containing matrix at any area ratio. Preferably, the ratio of the area of the drug-containing matrix to which the upper second liner adheres, to the area to which the lower V-shaped first liner adheres is in the range of 3:1 to 1:3, and more preferably in the range of 1:1 to 2:1.

If the ratio of the area to which the upper second liner adheres, to the area to which the lower V-shaped first liner adheres is smaller than 3:1, then the user needs to take special care to prevent the adhesive patch from falling during the application. Specifically, in applying the adhesive patch to the application site, the user first peels the upper second liner and attaches the drug-containing matrix to the application site. The user then peels the upper V-shaped first liner by slightly sliding the first liner with his fingers. During this, the user needs to keep the adhesive patch from falling by pressing the adhesive patch against the application site with the other hand. Furthermore, the lower V-shaped liner that adheres to a larger area leads to an increased cost.

Likewise, if the ratio of the area to which the upper second liner adheres, to the area to which the lower V-shaped first liner adheres is larger than 1:3, then the user must peel the liners slowly and carefully to keep the drug-containing matrix from sticking to itself during the removal of the upper second liner. In addition, the distance between the tab of the upper second liner and the tab of the lower V-shaped first liner becomes so small that the user, in an attempt to peel the upper second liner, may pinch both of the tabs of the upper and the lower liners at once.

The cut edge of the tab of the upper second liner may be shaped into any shape including, but not limited to, straight line, wave shape and mountain shape. While the end of the tab of the upper second liner may be shaped into any shape ranging from a straight line to a gently curved line, pointy shapes are not preferred since the user may cut his finger with the edge of the liner.

Likewise, the cut edge of the tab of the lower V-shaped first liner may also be shaped into any shape including, but not limited to, straight line, wave shape and mountain shape. While the end of the tab of the lower V-shaped first liner may be shaped into any shape ranging from a straight line to a gently curved line, pointy shapes are not preferred since the user may cut his finger with the edge of the liner. Pointy shapes are not preferred also because the pointy edges can interfere with the adhering of the lower V-shaped liner to the drug-containing matrix during the production of the adhesive patches, resulting in a decreased yield of the adhesive patches.

The backing for use in the adhesive patch of the present invention may be woven fabric, non-woven fabric or a laminate thereof and may or may not be stretchable.

Specific examples of the material for the backing include natural fibers, including bast fibers, such as paper, cotton, hemp and jute, cellulose fibers, such as leaf fibers (such as manila hemp), animal fibers, such as wool, and protein fibers, such as silk fibers and feather fibers; regenerated fibers, including regenerated cellulose fibers, such as rayon and cuprammonium rayon, and regenerated protein fibers; semi-synthetic fibers, including cellulose acetate fibers and promix; nylon alamide fibers; polyethylene terephthalate fibers; polyester fibers; acrylic fibers; polyolefin fibers, including polyethylene fibers and polypropylene fibers; polyvinyl alcohol fibers; polyvinyl chloride fibers; polyvinylidene chloride fibers; polyvinyl chloride-based fibers; polyurethane fibers; polyoxymethylene fibers; polytetrafluoroethylene fibers; poly(p-phenylene benz-bisthiazole) fibers; and polyimide fibers. These fibers may be used either individually or as a composite fiber to make a woven or non-woven fabric for use in the present invention.

The backing is properly selected from the above-described materials based on the tensile strength, thickness and stretchability required for a particular application site, as well as drug transfer to the backing.

The drug-containing matrix for use in the adhesive patch contains a base and a drug and is suitable for adhesive patches used, for example, as external poultice-type patches. The drug-containing matrix also contains water to enhance the effect of the drug on the skin. The drug-containing matrix has stickiness and does not soften at room temperature or a higher temperature so that it retains moderate cohesiveness that prevents the drug-containing matrix from remaining on the skin.

A thickener may be used in the drug-containing matrix. The thickener serves to stably maintain the water content of the drug-containing matrix at 30% to 80% and preferably shows water retention. Specific examples of the thickener include water-soluble polymers, including natural polymers, such as plant-based polymers (such as guar gum, locust bean gum, carrageenan, alginic acid, sodium alginate, agar, gum acacia, tragacanth gum, karaya gum, pectin and starch), microorganism-based polymers (such as xanthan gum and acacia gum), and animal-based polymers (such as gelatin and collagen); semi-synthetic polymers, such as cellulose-based polymers (such as methyl cellulose, ethyl cellulose, hydroxyethyl cellulose and carboxymethylcellulose sodium), and starch-based polymers (such as soluble starch, carboxymethyl starch and dialdehyde starch); and synthetic polymers, such as vinyl-based polymers (such as polyvinyl alcohol, polyvinylpyrrolidone and polyvinylmethacrylate), acryl-based polymers (such as polyacrylic acid and sodium polyacrylate), polyoxyethylene oxides, and methylvinylether/maleic anhydride copolymers.

Of these, sodium polyacrylate is particularly preferred because of its high gel strength and good water retention. Sodium polyacrylate preferably has an average degree of polymerization of 20,000 to 70,000. Sodium polyacrylate having an average degree of polymerization of less than 20,000 exhibits less thickening effect, failing to provide sufficient gel strength. Conversely, sodium polyacrylate exhibits too high a thickening effect and leads to decreased workability when it has an average degree of polymerization of higher than 70,000. An elastic gel having even higher gel strength can be obtained by using sodium polyacrylate in combination with two or more of the above-described water-soluble polymers since sodium polyacrylate, a highly ionic polymer, forms a polymer complex with the water-soluble polymers.

A humectant may be used in the drug-containing matrix. Examples include polyols, such as glycerin, propylene glycol and sorbitol. In addition, a filler, such as kaolin, zinc oxide, talc, titanium, bentonite, aluminum silicate, titanium oxide, zinc oxide, aluminum metasilicate, calcium sulfate and calcium phosphate, may be added. A solubilizer or an absorption enhancer, such as propylene carbonate, crotamiton, 1-menthol, mint oil, limonene and diisopropyl adipate, may also be added. A drug-activity enhancer, such as methyl salicylate, glycol salicylate, 1-menthol, thymol, mint oil, nonanoic acid vanillylamide and capsicum extract, may also be added. When necessary, a stabilizer, an antioxidant or an emulsifier may also be added.

When necessary, the drug-containing matrix may also contain a crosslinking agent or a polymerization agent to strengthen the drug-containing matrix and to impart the water retention property to the drug-containing matrix. The crosslinking agent or the polymerization agent may be properly selected depending on the type of the thickener used. For example, when the thickener is polyacrylic acid or a polyacrylate, the crosslinking agent is preferably a polyvalent metal compound, including compounds having at least two epoxy groups in their molecules; inorganic salts, such as hydrochlorides, sulfates, phosphates and carbonates of Ca, Mg and Al; organic salts, such as citrates, tartrates, gluconates and stearates; oxides, such as zinc oxide and silicic anhydride; and hydroxides, such as aluminum hydroxide and magnesium hydroxide.

When the thickener is polyvinyl alcohol, the crosslinking agent or the polymerization agent is preferably adipic acid, thioglycol acid, epoxy compounds (epichlorohydrin), aldehydes, N-methylol compounds or a complex of compounds, such as Al, Ti, Zr, Sn, V, Cu, B and Cr.

When the thickener is polyvinylpyrrolidone, the crosslinking agent or the polymerization agent is preferably a methylvinylether/maleic anhydride copolymer, or a polyacid compound or an alkali metal salt thereof (such as polyacrylic acid, tannic acid and derivatives thereof).

When the thickener is polyethylene oxide, the crosslinking agent or the polymerization agent is preferably a peroxide or polysulfone azide. When the thickener is methylvinylether/maleic anhydride copolymer, the crosslinking agent or the polymerization agent is preferably a polyfunctional hydroxyl compound, polyamine, iodine, gelatin, polyvinylpyrrolidone, iron, mercury or lead salt.

When the thickener is gelatin, the crosslinking agent or the polymerization agent is preferably an aldehyde, such as formaldehyde, glutaraldehyde and dialdehyde starch, a diepoxide, such as gluoxal and butadieneoxide, a diketone, such as divinyl ketone, or a diisocyanate. When the thickener is sodium polyacrylate, the crosslinking agent added is preferably a polyvalent metal salt, such as lithium hydroxide, zinc hydroxide, aluminum hydroxide and sodium borate. Zinc salts and aluminum salts promote crosslinking reactions and are thus particularly preferred.

The polyvalent metal salt used as the crosslinking agent is preferably used at a concentration of 0.5 to 1.5 equivalents relative to 1 equivalent of the thickener (or water-soluble polymer). The polyvalent metal salt used at a concentration of less than 0.5 equivalents does not sufficiently promote the crosslinking reaction, resulting in a low gel strength, whereas the polyvalent metal salt used at a concentration of higher than 1.5 equivalents excessively accelerates the crosslinking reaction, resulting in non-uniform gelation and, thus, decreased workability.

A poultice-type patch is required to stick firmly to the skin, enhance absorption of the active ingredient by the skin, and contain as much water as possible. The water present in the drug-containing matrix removes heat from the skin and causes coolness as it evaporates. The water molecules evaporating from inside the matrix hydrate the stratum corneum and thereby promote the drug absorption. Requirements for the drug-containing matrix include the following: it should not soften near room temperature; it should not cause pain or remain on the skin when the adhesive patch is peeled; and it should not cause stickiness.

To meet the above-described requirements, the drug-containing matrix contains 5 to 20 wt%, preferably 10 to 15 wt% of the thickener, 5 to 40 wt% of the humectant, 20 wt% or less of the filler, 10 to 80 wt% of water, 0 to 8 wt% of the solubilizer, and 5 wt% or less, preferably 0.5 to 5 wt% of the drug.

The drug used as an active ingredient in the adhesive patch of the present invention can be selected from a variety of drugs to suit the intended application. Analgesic and anti-inflammatory agents that can be used in the adhesive patch include indomethacin, ketoprofen, flurbiprofen, ibuprofen, felbinac, glycol salicylate, methyl salicylate, glycyrrhizinic acid, dipotassium glycyrrhizinate and β-glycyrrhizinic acid.

Blood circulation-promoting agents that can be used in the adhesive patch include tocopherol acetate, capsicum extract, capsaicin, nonanoic acid vanillylamide, benzyl nicotinate and benzyl alcohol. Antiallergic agents that can be used in the adhesive patch include diphenhydramine hydrochloride and chlorpheniramine maleate. Locally stimulating agents that can be used in the adhesive patch include 1-menthol, camphor, mint oil and eucalyptus oil. Local anesthetic agents that can be used in the adhesive patch include lidocaine, benzocaine, dibucaine and tetracaine. The drugs for use in the adhesive patch are not limited to those described above and may be used in combination of two or more as desired.

The amount of the drug used in the drug-containing matrix is properly determined depending on the type and the intended application of the adhesive patch such as poultice-type patch so that the drug is delivered to the application site at a predetermined effective dose when the adhesive patch is applied to the patients.

### Examples

The present invention will now be described by way of several embodiments serving as concrete examples with reference to the accompanying drawings.

### [Embodiment 1]

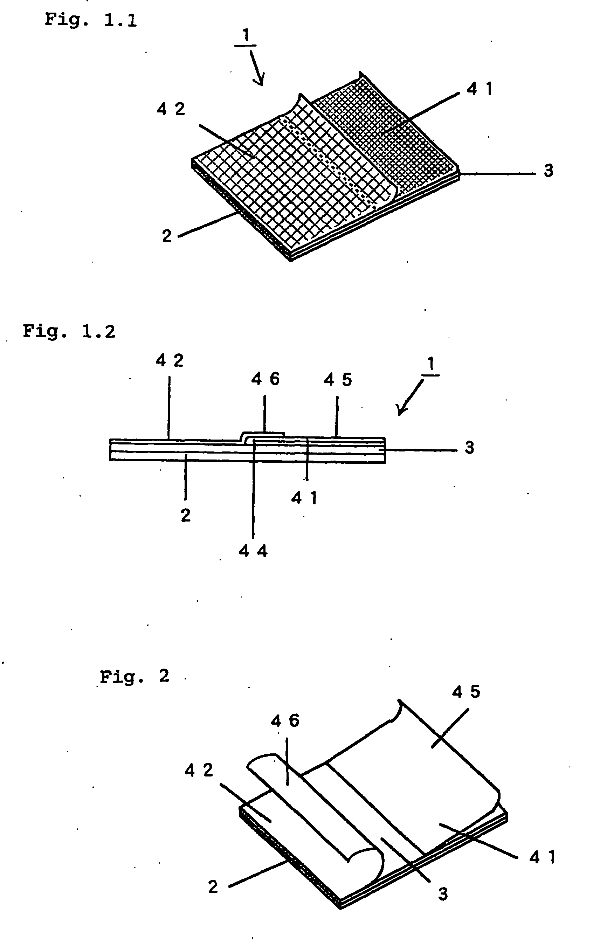

Shown in Fig. 1 is an adhesive patch, such as a poultice-type patch, of Embodiment 1 provided in accordance with one example of the present invention. Fig. 1.1 is a perspective view of the adhesive patch and Fig. 1.2 is a side view thereof.

The poultice-type patch 1 or the adhesive patch of example shown in Fig. 1 is essentially constructed as a laminate comprising a backing 2 formed of a stretchable non-woven fabric, a drug-containing matrix 3 spread over the substantially entire surface of the backing 2, and a pair of liners 41 and 42 that adheres to the surface of the drug-containing matrix 3.

Of the two liners that adhere to the surface of the drug-containing matrix 3 of the poultice-type patch 1, the lower first liner 41 is folded at the middle thereof so that it is divided by the fold into two sections that together form a V-shape. One of the two sections of the V-shaped first liner 41 adheres to the drug-containing matrix surface from one end of the matrix so that the fold 44 is arranged closer to the middle of the drug-containing matrix. The other of the two sections of the V-shaped liner serves as a tab 45 to be held by the user's fingers.

Of the two liners that adhere to the drug-containing matrix surface, the upper second liner 42 adheres to the remaining part of the drug-containing matrix surface so that one end of the second liner covers the fold 44 of the lower V-shaped first liner 41 to form a laminated part that serves as a tab 46.

In the present example, the drug-containing matrix 3 spread over the substantially entire surface of the backing 2 is formed of a material such as sodium polyacrylate and contains, along with water, a drug such as felbinac, an anti-inflammatory/analgesic agent that serves as an active ingredient.

In the present example, the liners 41 and 42 that adhere to the surface of the drug-containing matrix are each a 30 to 50µm-thick liner formed of cast polypropylene. The upper second liner 42 is embossed with a diamond pattern while the lower V-shaped first liner 41 is embossed with a hexagonal pattern.

In the present example, the ratio of the area of the drug-containing matrix 3 to which the upper second liner 42 adheres, to the area to which the lower V-shaped first liner 41 adheres is 1:1. The tab 46 in which the upper second liner 42 is laminated to the first liner is preferably 15 to 25 mm in width.

How the poultice-type patch 1 that is constructed based on the above-described example and serves as the adhesive patch of the present invention is applied to an application site will now be described.

Fig. 2 shows in a schematic perspective view the manner in which the upper second liner 42 of the poultice-type patch 1 of the above-described example is peeled.
Fig. 3 shows in a schematic side view the manner in which the drug-containing matrix 3 having substantially half of its surface exposed by peeling the upper second liner 42 of the poultice-type patch 1 of the above-described example is attached to the application site.
Fig. 4 shows in a schematic side view the manner in which the drug-containing matrix 3, following the procedure of Fig. 3, is continuously attached to the application site as the remaining lower V-shaped first liner 41 is peeled off.

Specifically, the user first pinches with his fingers (not shown) the tab 46 of the upper second liner 42 of the poultice-type patch 1 and peels the upper second liner 42 as shown in Fig. 2. Once the second liner 42 is completely peeled, (half of) the surface of the drug-containing matrix 3 to which the second liner 42 has adhered is exposed.

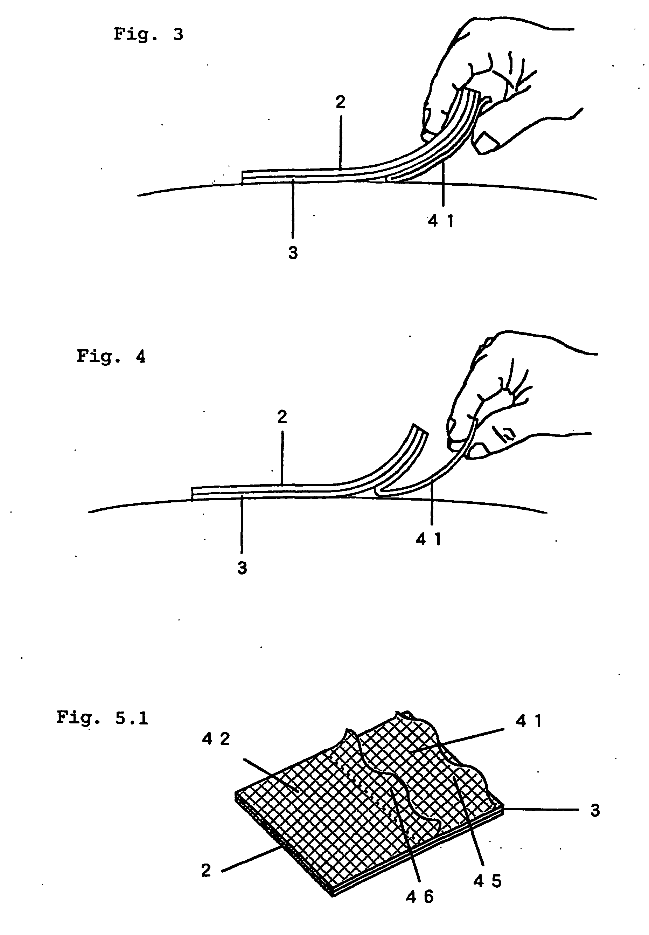

Subsequently, the user attaches the exposed surface of the drug-containing matrix to the application site and then holds the backing 2 together with the tab 45 of the lower V-shaped first liner 41, as shown in Fig. 3.

As shown in Fig. 4, the user then slides outward one of the fingers held against the tab 45 of the lower V-shaped first liner 41. The peeling of the first liner 41 causes the exposed drug-containing matrix 3 to spontaneously attach to the application site.

As a result, the series of actions required to apply the patch, starting from the removal of the liner and ending in the application of the poultice-type patch, can be readily carried out in a single sequence. In addition, the user can apply the poultice-type patch in a safe and hygienic manner without his hands coming into contact with the drug-containing matrix during the application.

### [Embodiment 2]

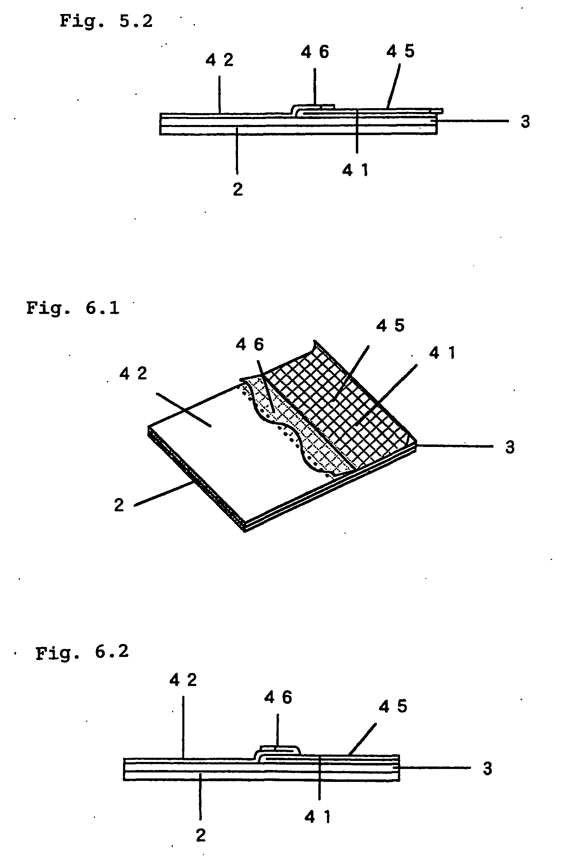

Shown in Fig. 5 is an adhesive patch, such as a poultice-type patch, of Embodiment 2 provided in accordance with another example of the present invention. Fig. 5.1 is a perspective view of the adhesive patch and Fig. 5.2 is a side view thereof.
The numerals in Fig. 5 denote the same elements as in Fig. 1.

In the present example, the poultice-type patch 1 is similar to the poultice-type patch of the above-described example in that it is constructed as a laminate comprising a backing 2, a drug-containing matrix 3 spread over the entire surface of the backing 2, and a pair of liners 41 and 42 that adheres to the surface of the drug-containing matrix 3. However, the two liners 41, 42 used in the present example are each formed of a 25 to 38pm-thick silicone-treated polyethylene terephthalate sheet having a diamond pattern embossed thereon.

Furthermore, each of the tabs 45, 46 of the two liners adhering to the surface of the drug-containing matrix 3 has its edge cut into a wave shape.

In the present example, the poultice-type patch 1 is applied to the application site in the same manner as in the above-described example. However, the two liners are formed of a 25 to 38µm-thick polyethylene terephthalate sheet that is harder than cast polypropylene used in the above-described example. Thus, if the edges of the tabs 46, 45 of the second and first liners, arranged one on top of the other, are shaped as a straight edge, the user can inadvertently injure his fingers while trying to pinch the tabs with his fingers.

To prevent this, the cut edges of the tabs 45, 46 in the present example are shaped into a wave shape to significantly reduce the risk of cutting fingers.
In other words, cutting the edge of each of the tabs 45, 46 into a wave shape as in this example can prevent cuts and other injuries to the fingers and thus ensure safe peeling of the films even when a hard material is used to make the two liners that are arranged one on top of the other.
It should be understood that the edge of each of the tabs 45, 46 may be shaped not only as the wave shape employed in this example, but also as a mountain shaped curved line or a combination thereof.

### [Embodiment 3]

Shown in Fig. 6 is an adhesive patch, such as a poultice-type patch, of Embodiment 3 provided in accordance with still another example of the present invention. Fig. 6.1 is a perspective view of the adhesive patch and Fig. 6.2 is a side view thereof.
The numerals in Fig. 6 denote the same elements as in Fig. 1.

In this example, the poultice-type patch 1 is similar to the poultice-type patch of the above-described example in that it is constructed as a laminate comprising a backing 2, a drug-containing matrix 3 spread over the entire surface of the backing 2, and a pair of liners 41, 42 that adheres to the surface of the drug-containing matrix 3. However, the two liners 41, 42 used in the present example are each formed of a 12µm-thick polyethylene terephthalate sheet. The lower V-shaped first liner has a diamond pattern embossed thereon and the upper second liner is not embossed.

Furthermore, the tab 46 of the upper second liner 42 has its edge cut into a wave shape and is folded 180 degrees to form a multilayer structure.

In the present example, the poultice-type patch 1 is applied to the application site in essentially the same manner as in the above-described example. The 12µm-thick polyethylene terephthalate sheet used to make the two liners in the present example has a relatively low hardness and poses a small risk of injuries to the fingers. Nonetheless, the thin film makes it difficult for the user to hold the tab 46 of the upper second liner 42. To cope with this, the tab 46 is folded 180 degrees to form a multilayer structure that helps the user to pinch the tab 46 with his fingers. In addition, the cut edge of the tab 46 is shaped into a wave shape to provide a grip that facilitates holding with fingers.

Furthermore, the upper second liner 42 without any embossed pattern formed on it allows the user to easily distinguish between the two upper and lower liners 42, 41 both visually and by touching.
In other words, the poultice-type patch in the present example enables the user to easily distinguish the tabs and peel the films in a safe and accurate manner even when a less hard material is used to make the two liners 42, 41 that are arranged one on top of the other. This is possible by the tab 46 of the upper second liner 42 that is folded 180 degrees and has its edge cut into a wave shape, in conjunction with the upper and lower liners that are embossed differently.

### INDUSTRIAL APPLICABILITY

As set forth, the adhesive patch of the present invention uses two liners, first and second liners, that adhere to the surface of the drug-containing matrix and each include a tab that the user can easily pinch with his fingers to peel the films. This construction not only allows the user to remove the liners in a safe and simple manner by exerting only a weak force, but also enables the user to easily perform, with a single hand, the sequence of actions required to apply the patch, from the removal of the liners to the application of the patch.

According to the adhesive patch of the present invention, the stickiness and the skin followability of the drug-containing matrix are not affected since the drug-containing matrix does not come into contact with the hands or fingers during the sequence of actions from the removal of the liner to the application of the patch to the application site. Since the drug-containing matrix is free of contamination, the adhesive patch can be applied in a highly hygienic manner and is therefore of significant industrial importance.

### BRIEF DESCRIPTION OF THE DRAWINGS

Fig. 1.1 is a perspective view showing a poultice-type patch 1 of Embodiment 1 provided in accordance with one example of the present invention.
Fig. 1.2 is a side view of the poultice-type patch 1 of Embodiment 1.
Fig. 2 is a schematic perspective view showing the manner in which the upper second liner 42 of the poultice-type patch 1 of the above-described example of the present invention is peeled.
Fig. 3 is a schematic side view showing the manner in which the upper second liner 42 of the poultice-type patch 1 of the above example of the present invention is peeled to expose substantially half of the surface of the drug-containing matrix 3 to apply the patch to the application site.
Fig. 4 is a schematic side view showing the manner in which the drug-containing matrix 3, following the procedure of Fig. 3, is continuously attached to the application site as the remaining lower V-shaped first liner 41 is peeled off.

Fig. 5.1 is a perspective view showing a poultice-type patch of Embodiment 2 provided in accordance with another example of the present invention.
Fig. 5.2 is a side view of the poultice-type patch 1 of Embodiment 2.
Fig. 6.1 is a perspective view showing a poultice-type patch 1 of Embodiment 3 provided in accordance with still another example of the present invention.
Fig. 6.2 is a side view of the poultice-type patch 1 of Embodiment 3.
Fig. 7.1 is a perspective view showing a conventional poultice-type patch.
Fig. 7.2 is a side view of the conventional poultice-type patch.
Fig. 8.1 is a perspective view showing another conventional poultice-type patch.

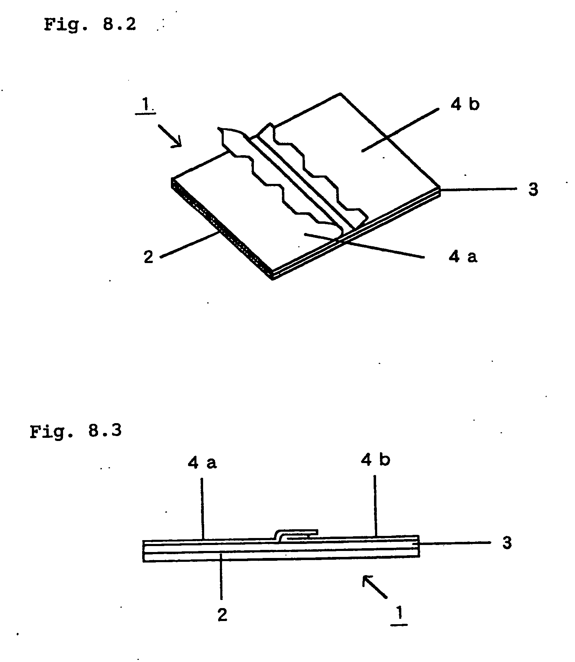

Fig. 8.2 is a perspective view showing the conventional poultice-type patch that has its peelable paper sheet removed.
Fig. 8.3 is a side view of the conventional poultice-type patch.

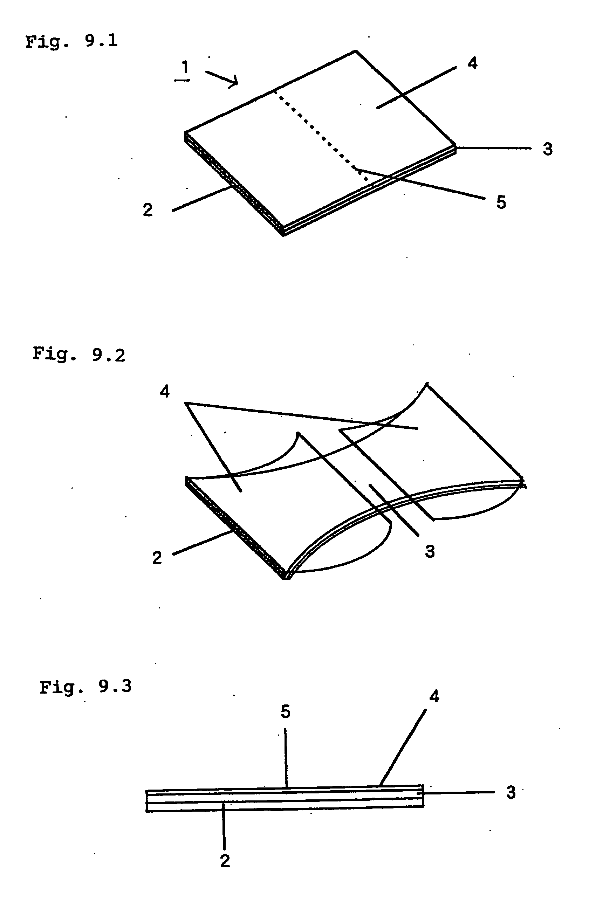

Fig. 9.1 is a perspective view showing another conventional poultice-type patch.
Fig. 9.2 is a perspective view showing the conventional poultice-type patch with its backing stretched.
Fig. 9.3 is a side view of the conventional poultice-type patch.

### DESCRIPTION OF REFERENCE NUMERAL

- 1: poultice-type patch (adhesive patch)
- 2: backing
- 3: drug-containing matrix
- 4: liner
- 41: first liner
- 42: second liner
- 44: fold
- 45: tab
- 46: tab

## Claims

1. An adhesive patch having a multilayer structure comprising a backing (2), an adhesive drug-containing matrix (3) that is spread substantially entirely over one surface of the backing (2), and a liner (4) that adheres to the drug-containing matrix (3) surface, wherein the adhesive drug-containing matrix comprises sodium polyacrylate as thickener, and wherein
(a) the liner (4) adhering to the drug-containing matrix surface comprises first (41) and second liners (42);
(b) the first liner (41) is folded at a middle thereof so that it is divided by the fold (44) into first and second sections that together form a V-shaped liner, the first section adhering to the drug-containing matrix surface from one end of the matrix surface with the fold arranged closer to the middle of the drug-containing matrix (3), the second section serving as a tab (45, 46) of the V-shaped liner, wherein the tab (45, 46) of the first liner (41) and/or the second liner (42) has an end shaped as a curved line, such as a wave shape or a mountain shape, or a combination thereof; and
(c) the second liner (42) adheres to the remaining part of the drug-containing matrix (3) surface with one end of the second liner (42) covering the fold of the V-shaped first liner (41) to form a laminated part that serves as a tab (45, 46);
and, further wherein the ratio of an area of the drug-containing matrix (3) surface to which the first liner (41) adheres, to an area to which the second liner (42) adheres is in a range of 3:1 to 1:3.

2. The adhesive patch according to claim 1, wherein the ratio of an area of the drug-containing matrix (3) surface to which the first liner (41) adheres to an area to which the second liner (42) adheres is in a range of 1:1 to 2:1.

3. The adhesive patch according to any of claims 1 to 2, wherein the tab (45, 46) of the second liner (42) is 10 mm to 30 mm.

4. The adhesive patch according to any of claims 1 to 3, wherein the first liner (41) and/or the second liner (42) comprises a plastic film formed of cast polypropylene, oriented polypropylene, polyethylene terephthalate, polybutylene terephthalate, polyethylene, polyester, polyurethane, polyvinyl chloride or polystyrene; paper, synthetic paper, synthetic resin or a composite film formed of a laminate thereof; or a composite film formed of a laminate of aluminum foil or aluminum-deposited film.

5. The adhesive patch according to any of claims 1 to 4, wherein the first liner (41) and/or the second liner (42) is treated with silicone.

6. The adhesive patch according to any of claims 1 to 5, wherein the first liner (41) and/or the second liner (42) is embossed.

7. The adhesive patch according to any of claims 1 to 6, wherein the tab (45, 46) of the first liner (41) and/or the second liner (42) has a decorative pattern or is colored.

8. The adhesive patch according to any of claims 1 to 6, wherein the tab (45, 46) of the first liner (41) and/or the second liner (42) is embossed differently from the remaining part of the film.

## Patentansprüche

1. Klebepflaster, das eine Mehrschichtstruktur hat, die einen Träger (2), eine klebende Arzneimittel-enthaltende Matrix (3), die im Wesentlichen über eine ganze Oberfläche des Trägers verteilt ist, und eine Trennlage (4), die an der Arzneimittel-enthaltenden Matrix (3)-Oberfläche klebt, umfasst, wobei die klebende Arzneimittel-enthaltende Matrix Natriumpolyacrylat als Verdickungsmittel umfasst und wobei
(a) die Trennlage (4), die an der Arzneimittelenthaltenden Matrix-Oberfläche klebt, eine erste (41) und eine zweite Trennlage (42) umfasst;
(b) die erste Trennlage (41) in der Mitte gefalzt ist, sodass sie durch den Falz (44) in einen ersten und einen zweiten Abschnitt geteilt wird, die zusammen eine V-förmige Trennlage bilden, wobei der erste Abschnitt, der an der Arzneimittel-enthaltenden Matrix-Oberfläche klebt, von einem Ende der Matrix-Oberfläche mit dem Falz näher an der Mitte der Arzneimittel-enthaltenden Matrix (3) angeordnet ist, der zweite Abschnitt als eine Lasche (45, 46) der V-förmigen Trennlage dient, wobei die Lasche (45, 46) der ersten Trennlage (41) und/oder der zweiten Trennlage (42) ein Ende hat/haben, das als gekrümmte Linie geformt ist, zum Beispiel eine Wellenform oder eine Bergform oder eine Kombination davon, und
(c) die zweite Trennlage (42) an dem restlichen Teil der Arzneimittel-enthaltenden Matrix (3)-Oberfläche klebt, wobei ein Ende der zweiten Trennlage (42) den Falz der V-förmigen ersten Trennlage (41) bedeckt, um einen laminierten Teil zu bilden, der als eine Lasche (45, 46) dient,
und wobei außerdem das Verhältnis einer Fläche der Arzneimittel enthaltenden Matrix (3)-Oberfläche, an der die erste Trennlage (41) klebt, zu einer Fläche, an der die zweite Trennlage (42) klebt, in einem Bereich von 3:1 bis 1:3 liegt.

2. Klebepflaster gemäß Anspruch 1, wobei das Verhältnis einer Fläche der Arzneimittel-enthaltenden Matrix (3)-Oberfläche, an der die erste Trennlage (41) klebt, zu einer Fläche, an der die zweite Trennlage (42) klebt, in einem Bereich von 1:1 bis 2:1 ist.

3. Klebepflaster gemäß einem der Ansprüche 1 bis 2, wobei die Lasche (45, 46) der zweiten Trennlage (42) 10 mm bis 30 mm ist.

4. Klebepflaster gemäß einem der Ansprüche 1 bis 3, wobei die erste Trennlage (41) und/oder die zweite Trennlage (42) einen Kunststofffilm, gebildet aus gegossenem Polypropylen, orientiertem Polypropylen, Polyethylenterephthalat, Polybutylenterephthalat, Polyethylen, Polyester, Polyurethan, Polyvinylchlorid oder Polystyrol; Papier, synthetischem Papier, synthetischem Harz oder einen Verbundfilm, gebildet aus einem Laminat davon, oder einen Verbundfilm, gebildet aus einem Laminat aus Aluminiumfolie oder einem Film mit abgeschiedenem Aluminium, umfassen/umfasst.

5. Klebepflaster gemäß einem der Ansprüche 1 bis 4, wobei die erste Trennlage (41) und/oder die zweite Trennlage (42) mit Silicon behandelt sind/ist.

6. Klebepflaster gemäß einem der Ansprüche 1 bis 5, wobei die erste Trennlage (41) und/oder die zweite Trennlage (42) geprägt sind/ist.

7. Klebepflaster gemäß einem der Ansprüche 1 bis 6, wobei die Lasche (45, 46) der ersten Trennlage (41) und/oder der zweiten Trennlage (42) ein dekoratives Muster haben/hat oder gefärbt sind/ist.

8. Klebepflaster gemäß einem der Ansprüche 1 bis 6, wobei die Lasche (45, 46) der ersten Trennlage (41) und/oder der zweiten Trennlage (42) anders geprägt sind/ist als der verbleibende Teil des Films.

## Revendications

1. Timbre adhésif ayant une structure multicouche comprenant un support (2), une matrice adhésive contenant un médicament (3) qui est étalée substantiellement entièrement sur une surface du support (2) et un revêtement (4) qui adhère à la surface de la matrice contenant un médicament (3), la matrice adhésive contenant un médicament comprenant du polyacrylate de sodium comme épaississant, et dans lequel
(a) le revêtement (4) adhérant à la surface de la matrice contenant un médicament comprend des premier (41) et deuxième revêtements (42) ;
(b) le premier revêtement (41) est replié en son milieu de sorte qu'il est divisé par le pli (44) en première et deuxième parties qui forment ensemble un revêtement en forme de V, la première partie adhérant à la surface de la matrice contenant un médicament, d'une extrémité de la surface de la matrice, le pli étant disposé à proximité du milieu de la matrice contenant un médicament (3), la deuxième partie servant de languette (45, 46) du revêtement en forme de V, la languette (45, 46) du premier revêtement (41) et/ou du deuxième revêtement (42) ayant une extrémité formée comme une ligne courbe telle qu'une forme de vague ou une forme de montagne ou une combinaison de celles-ci ; et
(c) le deuxième revêtement (42) adhère à la partie restante de la surface de la matrice contenant un médicament (3), une extrémité du deuxième revêtement (42) couvrant le pli du premier revêtement en forme de V (41) pour former une partie stratifiée qui sert de languette (45, 46) ;
et, en outre dans lequel le rapport d'une surface de la surface de la matrice contenant un médicament (3) à laquelle le premier revêtement adhère (41), à une surface à laquelle le deuxième revêtement (42) adhère est de l'ordre de 3:1 à 1:3.

2. Timbre adhésif selon la revendication 1, dans lequel le rapport d'une surface de la surface de la matrice contenant un médicament (3) à laquelle le premier revêtement (41) adhère, à une surface à laquelle le deuxième revêtement (42) adhère est de l'ordre de 1:1 à 2:1.

3. Timbre adhésif selon l'une quelconque des revendications 1 à 2, dans lequel la languette (45, 46) du deuxième revêtement (42) est de 10 mm à 30 mm.

4. Timbre adhésif selon l'une quelconque des revendications 1 à 3, dans lequel le premier revêtement (41) et/ou le deuxième revêtement (42) comprennent un film plastique formé de polypropylène coulé, de polypropylène orienté, de polyéthylène téréphtalate, de polybutylène téréphtalate, de polyéthylène, de polyester, de polyuréthane, de chlorure de polyvinyle ou de polystyrène ; de papier, de papier synthétique, de résine synthétique ou d'un film composite formé d'un stratifié de celui-ci ; ou d'un film composite formé d'un stratifié de feuille d'aluminium ou d'un film déposé sur aluminium.

5. Timbre adhésif selon l'une quelconque des revendications 1 à 4, dans lequel le premier revêtement (41) et/ou le deuxième revêtement (42) est traité avec de la silicone.

6. Timbre adhésif selon l'une quelconque des revendications 1 à 5, dans lequel le premier revêtement (41) et/ou le deuxième revêtement (42) est gaufré.

7. Timbre adhésif selon l'une quelconque des revendications 1 à 6, dans lequel la languette (45, 46) du premier revêtement (41) et/ou du deuxième revêtement (42) a un motif décoratif ou est colorée.

8. Timbre adhésif selon l'une quelconque des revendications 1 à 6, dans lequel la languette (45, 46) du premier revêtement (41) et/ou du deuxième revêtement (42) est gaufrée de façon différente de la partie restante du film.
